(19) Europäisches Patentamt
European Patent Office
Office européen des brevets

(11) EP 1 476 019 B1

(12) # EUROPEAN PATENT SPECIFICATION

(45) Date of publication and mention of the grant of the patent:
**02.04.2008 Bulletin 2008/14**

(21) Application number: **03739600.9**

(22) Date of filing: **04.02.2003**

(51) Int Cl.:
***A01N 43/40*** *(2006.01)*

(86) International application number:
**PCT/IB2003/000351**

(87) International publication number:
**WO 2003/067988 (21.08.2003 Gazette 2003/34)**

(54) **AQUEOUS ANTISEPTIC BASED ON BISPYRIDINIUMALKANES**

WÄSSRIGES ANTISEPTIKUM AUF DER BASIS VON BISPYRIDINIUMALKANEN

ANTISEPTIQUE AQUEUX A BASE DE BISPYRIDINIUMALKANES

(84) Designated Contracting States:
**AT BE BG CH CY CZ DE DK EE ES FI FR GB GR HU IE IT LI LU MC NL PT SE SI SK TR**

(30) Priority: **13.02.2002 DE 10205883**

(43) Date of publication of application:
**17.11.2004 Bulletin 2004/47**

(73) Proprietors:
- **Air Liquide Santé (International)**
  **75341 Paris Cedex 07 (FR)**
  Designated Contracting States:
  **AT BE BG CH CY CZ DK EE ES FI FR GB GR HU IE IT LI LU MC NL PT SE SI TR**
- **Schulke & Mayr**
  **22851 Norderstedt (DE)**
  Designated Contracting States:
  **DE**

(72) Inventors:
- **BEHRENDS, Sabine**
  **25421 Pinneberg (DE)**
- **GORONCY-BERMES, Peter**
  **22145 Hamburg (DE)**
- **PUCHSTEIN, Burghard**
  **20257 Hamburg (DE)**
- **SIEBERT, Jörg**
  **23843 Bad Oldesloe (DE)**

(74) Representative: **Conan, Philippe Claude**
**L'Air Liquide SA**
**Département Propriété Intellectuelle**
**75, quai d'Orsay**
**75321 Paris Cédex 07 (FR)**

(56) References cited:
**WO-A-98/20095**
**WO-A-98/53036**
**DE-A- 2 708 331**
**US-A- 4 420 484**

EP 1 476 019 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to an aqueous, isotonically adjustable antiseptic based on bispyridiniumalkanes, in particular on octenidine dihydrochloride (1,1'-decamethylenebis[1,4-dihydro-4-(octylimino)pyridine] dihydrochloride), and the use of the aqueous antiseptic for rendering skin, mucous membrane, wounds or internal organs antiseptic.

Antiseptics are employed before surgical interventions, before injections, punctures and before inspections of hollow organs when the skin or the mucous membrane has to be disinfected. In addition, antiseptics are also employed for wound treatment (surgical wounds, chronic wounds, burn wounds, bite wounds, cut wounds and traumatogenic wounds) and for the therapy of local superficial skin infections (e.g. in fungal infections). Irrigation (e.g. bladder and abdominal irrigation) is also carried out using antiseptics. Special application areas are additionally prophylactic, preoperative and therapeutic ophthalmic antisepsis and antisepsis of the oral cavity before maxillary surgical interventions and tooth extractions, for caries prophylaxis and in infections in the neck and pharingeal space.

The antiseptics previously known for the application areas mentioned have disadvantages in use for toxicological and/or ecological reasons or because of their inadequate activity. This applies especially to products containing the active compounds polyvinyl pyrrolidone-iodine (PVP-iodine), phenylmercury salts, tauroline, chlorhexidine, benzalkonium chloride or $H_2O_2$. The antibiotics employed especially for the therapy of local skin infections are only suitable to a restricted extent on account of possible development of resistance.

DE 27 08 331 C2 describes various bis[4-(substituted amino)-1-pyridinium]alkanes (in brief bispyridiniumalkanes), and a bacteriostatic activity and dental plaque-preventing activity of some representatives of this class of substance. The substance class consisting of the bispyridiniumalkanes in particular includes octenidine dihydrochloride.

Compositions based on octenidine dihydrochloride are known, and they are used for wound and suture care, and for hygienic and surgical hand disinfection. The antimicrobial action commences rapidly and lasts for a long time.

EP 0 411 315 A1 discloses an aqueous antiseptic composition which is suitable, in particular, as a mucous membrane antiseptic and for wound treatment and has a content of octenidine dihydrochloride and phenoxyethanol (POE) and/or phenoxypropanol (POP) in a weight ratio of octenidine to POE or POP of 1:20 to 1:11 at an octenidine dihydrochloride concentration of 0.005 to 0.5% by weight and a POE or POP concentration of 5 to 0.5% by weight. Furthermore, mixtures with various surfactants, for example cocosaminopropylbetaine, fatty acid monoglyceride, ethoxylated fatty alcohol polyglycol ether and polyoxyethylene fatty acid monoglyceride are disclosed

EP 0 161 425 and WO 98/20095 also disclose octenidine dihydrochloride-containing compositions and their use for the treatment of surfaces, including human skin. The aqueous compositions disclosed there can furthermore contain alcohols and surfactants.

DE 196 47 692 A1 discloses a hand washing disinfectant which comprises an aqueous solution which contains octenidine dihydrochloride, $C_1$- to $C_9$-alkyl alcohol, non-ionic and/or cationic surfactant and skin-compatible $\alpha$-hydroxycarboxylic acid.

Until now, however, it has still not been possible to prepare isotonic solutions based on bispyridinium-alkane, in particular octenidine dihydrochloride. Isotonicity is frequently desirable, a (strongly) diluted antimicrobial isotonic solution being demanded, inter alia, for irrigation of internal organs and washing of wounds. Attempts to isotonically adjust known compositions based on octenidine dihydrochloride showed that, after addition of salt, precipitation occurs. The dilution of the compositions mentioned with isotonic saline or Ringer's solution can also lead, especially at low temperatures, to precipitation, which is disadvantageous, particularly in the dispatch of such diluted solutions. Generally, hypo- or hypertonic solutions can lead to a reduction in the compatibility of the antiseptic. Cloudy compositions from which, under certain circumstances, a sediment deposits even on short-term storage are accordingly not suitable for use.

The invention was thus based on the object of making available an antiseptic based on bispyridiniumalkane, in particular octenidine dihydrochloride, which is both isotonically adjustable and, independently of whether it was isotonically adjusted or not, can be diluted with salt solutions to give isotonic solutions without precipitation occurring. There is always a need here for compositions which exhibit an improved activity with the same amount of active compound.

It has now surprisingly been found that this object is achieved by an alcohol-free, aryloxy alcohol-free isotonic antiseptic which comprises

a) 0.01 to 10% by weight of bispyridiniumalkane, in particular octenidine dihydrochloride, and

b) 0.01 to 20% by weight of non-ionic surfactant selected from alcohol polyalkoxylates, polysorbates and alkyl glycosides,

where the antiseptic can optionally contain one or more excipients.

Preferred embodiments are the subject of the subclaims.

For the purposes of the description of the present invention, the term "alcohol-free" means that the antiseptic according to the invention contains no monohydroxyalkyl alcohols, for example is free of the $C_1$- to $C_9$-alkyl alcohols

ethanol, propan-1-ol and propan-2-ol disclosed in DE 196 47 692 A1.

The term "aryloxy alcohol-free" means that the antiseptic according to the invention contains no aryloxy alcohols, for example the phenoxyalkyl alcohols phenoxyethanol and phenoxypropanol disclosed in EP 0 4.11 31.5 A1.

The term bispyridiniumalkane comprises the bis[4-(substituted amino)-1-pyridinium]alkanes disclosed in DE 27 08 331, of the general formula (I) or (II)

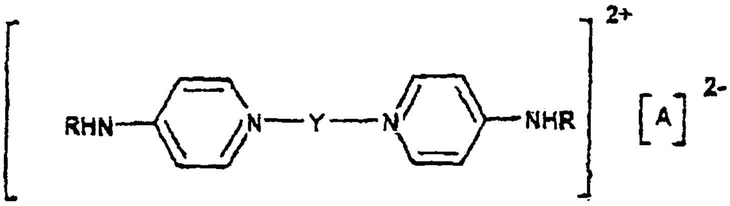 (I)

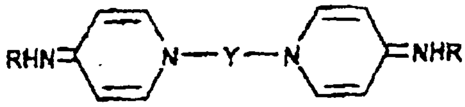 (II)

in which

Y is an alkylene group having 4 to 18 carbon atoms,

R is an alkyl group having 6 to 18 carbon atoms or a cycloalkyl group having 5 to 7 carbon atoms or the phenyl radical, which is substituted by a halogen atom, and

A is an anion or a number of anions.

The abovementioned definition for A taken strictly applies to mono- and divalent anions, but A can of course also be a polyvalent anion, e.g. phosphate or orthosilicate.

The term bispyridiniumalkane furthermore comprises the various prototropes of the compounds of the formula (I), as disclosed, for example, in DE 196 47 692 A1.

While all information for bispyridiniumalkanes applies to the entire substance class, it applies in particular to the octenidine dihydrochloride preferred in all embodiments of the invention.

The invention is based, inter alia, on the fact that it has surprisingly been found that antiseptics based on bispyridiniumalkane, in particular octenidine dihydro-chloride, and non-ionic surfactant selected from alcohol polyalkoxylates, polysorbates and alkyl glycosides can be isotonically adjusted and/or diluted with isotonic solutions if they are alcohol-free and aryloxy alcohol-free.

Preferred concentration ranges are 0.01 to 2% by weight of bispyridiniumalkane and 0.05 to 5% by weight of non-ionic surfactant, particularly preferably 0.02 to 0.5% by weight of bispyridiniumalkane and 0.1 to 2% by weight of non-ionic surfactant.

The alcohol polyalkoxylates include fatty alcohol alkoxylates, e.g. isodecyl ethoxylates containing various proportions of ethylene oxide, isotridecyl ethoxylates, polyethylene glycol ethers of stearyl alcohol, lauryl alcohol and cetyl alcohol and oleyl alcohol. In this case, the alcohols can have been alkoxylated with ethylene oxide, propylene oxide or any desired mixtures of ethylene oxide and propylene oxide. Alcohol polyalkoxylates are known, inter alia, under the names Lutensol®, Marlipal®, Marlox®, Brij® and Plurafac®.

Furthermore, non-ionic surfactants employed are the sorbitan esters usually present as oleates, stearates, laurates and palmitates, which are called polysorbates (e.g. Tween®).

Preferably, the non-ionic surfactant is an alkyl glycoside such as an alkyl glucoside (i.e. an alkyl glycoside of glucose), more preferably a $C_1$- to $C_{20}$-alkyl polyglucose, in particular a $C_8$- to $C_{12}$-alkyl polyglucose, a lauryl polyglucose, a decyl polyglucose or a mixture thereof being preferred. In the case of cocoyl polyglucose, the C chain length is 8 to 16 atoms, in the case of lauryl polyglucose 12 to 16 C atoms and in the case of decyl polyglucose likewise 8 to 16 C atoms. A particularly preferred non-ionic surfactant employed is the lauryl polyglucose marketed under the name Plantacare® 1200.

The antiseptic according to the invention is preferably free of anionic surfactants and amphosurfactants. Antiseptics are particularly preferred which contain- no cationic, anionic and amphosurfactants. The antiseptics furthermore preferably contain no amine oxides (which are sometimes employed as non-ionic surfactants) as the danger of the formation of nitrosamines is associated with these.

In addition to bispyridiniumalkane, in particular octenidine dihydrochloride, and non-ionic surfactant selected from alcohol polyalkoxylates, polysorbates and alkyl glycosides, an antiseptic according to the invention can optionally contain one or more excipients selected from dermoprotective substances such as allantoin, humectants such as glycerol, thickeners such as hydroxyethylcellulose and other cellulose derivatives, taste corrigents (for example when used as an oral antiseptic), refatting agents such as polyol fatty acid esters or lauryl alcohol containing 2 mol of EO, buffer substances, antifoam agents and colourants. According to the invention, particularly preferred excipients are sodium gluconate and glycerol (85% strength in water).

Preferably, the antiseptic according to the invention is present in the form of an isotonic solution. The isotonicity of the blood is about 290 mos/mol/kg (this corresponds to a 0.9% strength sodium chloride solution). The isotonicity can be adjusted using the appropriate amount of sodium chloride or, if an isotonic solution according to Ringer is desired, using 8.60 g/l of sodium chloride, 0.30 g/l of potassium chloride and 0.33 g/l of calcium chloride. 2 $H_2O$.

On the other hand, an antiseptic according to the invention, independently of whether it itself is present as an isotonic solution, can be diluted with salt solution, for example NaCl or Ringer's solution, such that the antiseptic solution obtained by dilution is isotonic. If the antiseptic according to the invention is an isotonic solution, then this solution will be carried out with isotonic dilution solution, for example isotonic NaCl or Ringer's solution. If the antiseptic according to the invention is not present as an isotonic solution, the dilution can correspondingly be carried out using a relatively highly concentrated salt solution, for example relatively highly concentrated NaCl or relatively highly concentrated Ringer's solution. The dilution of an isotonic antiseptic according to the invention is preferably carried out using an isotonic solution, namely in a volume ratio of isotonic antiseptic:isotonic solution in the range from 1:0.5 to 1:10, in particular in the volume ratio 1:1.

The antiseptics according to the invention have the following advantages:

► As shown by example of octenidine dihydrochloride-based antiseptics, these have an excellent antimicrobial activity; the antiseptics according to the invention are at least as active as known formulations of the active compound bispyridiniumalkane (at the same concentration).

► The antiseptics, independently of whether they have been isotonically adjusted or not, are clear solutions and remain clear even on storage at temperatures from 8°C to 40°C over a period of 60 months.

► The antiseptics, independently of whether they have already been isotonically adjusted or not, can be diluted with salt solutions, for example NaCl or Ringer's solution, to give isotonic solutions. The isotonic solutions obtained are clear and remain clear even on storage at temperatures from 8°C to 40°C over a period of 60 months.

► The antimicrobial activity is not influenced by the addition of excipients; turbidity also does not occur on addition of customary excipients.

► The absorption of alcohols and aryloxy alcohols is excluded, whereby possible complications in use are avoided from the start.

► By means of the presence of only one antimicrobial active compound, a simplification of application processes for licensing as medicaments results, particularly in the international market.

The advantages of the present invention are elucidated by the following examples.

Examples

The isotonicity of solutions was adjusted with the aid of an osmometer (semi-osmometer from Knaur). The measurement is carried out with this apparatus via the determination of the lowering of freezing point.

The turbidity measurements were carried out using the "Hach turbidimeter 2100AN" apparatus.

The determination of the activity was carried out in the following examples according to the test described under Example A.

A: Activity tests

The compositions of the antiseptics tested were as follows (details in % by weight):

| Component | Comparison antiseptic A | Comparison antiseptic B | Antiseptic 1 |
|---|---|---|---|
| Octenidine dihydrochloride | 0.10 | 0.10 | 0.10 |
| Phenoxyethanol | 2.00 | - | - |
| Cocoamidopropyl betaine (30% strength*) | 1.00 | 1.00 | - |
| Plantacare® 1200 (50% strength*) | - | - | 0.60 |
| Sodium gluconate | 0.40 | 0.40 | 0.40 |
| Glycerol (85% strength*) | 0.50 | 0.50 | 0.50 |
| Completely demineralized water | to 100.00 | to 100.00 | to 100.00 |
| * percentage by weight in water | | | |

The abovementioned surfactant-containing, octenidine dihydrochloride-containing, aqueous antiseptics were investigated for their microbicidal activity using *Staphylococcus aureus (S. aureus), Pseudomonas aeruginosa (P. aeruginosa)* and *Candida albicans (C. albicans),* the determination of the action being carried out according to the guidelines of the German Society for Hygiene and Microbiology (DGHM), dated 12.7.1991. The antiseptics 1, A and B differed, besides the presence or absence of phenoxyethanol, only in the type of surfactant, the pure surfactant content remaining the same. The times of action of the solutions prepared from the antiseptics 1 were 30 seconds, 1 minute, 2 minutes and 5 minutes. The application concentrations of the antiseptics tested were 0.25, 0.5, 0.75, 1.5, 3.125, 6.25, 12.5, 25, 50 and 100% by weight in water of standardized hardness.

The experiments were carried out as follows:

0.1 ml of the microorganism suspension in CSL (casein peptone-soya bean meal peptone solution) was mixed well at room temperature with 10 ml of the antiseptics (or the antiseptic dilutions present in the abovementioned concentrations). After the times of action mentioned had elapsed, in each case 1 ml of the antiseptic (dilution)/microorganism mixtures was withdrawn and inoculated into 9 ml of neutralization solution (0.1% by weight tryptone, 0.85% by weight NaCl, 3% by weight Tween® 80, 3% by weight saponin, 0.3% by weight lecithin, 0.1% by weight histidine in double-distilled water). Microorganism count determinations were then carried out in order to determine the reduction factors (see guideline of the DGHM of 12.7.1991).

The action of the test solutions was calculated according to the following formula:

$$KR = \log_{KBE(Ko)} - \log_{KBE(P)},$$

where

| | |
|---|---|
| $KBE_{(Ko)}$ | is the number of microorganisms/ml without action of the antiseptics (dilutions) mentioned, |
| $KBE_{(P)}$ | is the number of microorganisms/ml with action of the antiseptics (solutions) mentioned and |
| KR | is the logarithmic reduction factor. |

In the following tables, the activity is indicated as a function of time of action and antiseptic dilution (' = minutes, " = seconds):

**Table 1**

| Activity of antiseptics on *S. aureus* | | | | | | | | | | | | |
|---|---|---|---|---|---|---|---|---|---|---|---|---|
| | A | | | | B | | | | 1 | | | |
| | 30" | 1' | 2' | 5' | 30" | 1' | 2' | 5' | 30" | 1' | 2' | 5' |
| 0.250 | 0.00 | 0.00 | 0.00 | 0.00 | 0.00 | 0.00 | 0.00 | 0.00 | 0.00 | 0.00 | 0.00 | 0.00 |
| 0.500 | 0.00 | 0.00 | 0.00 | 0.00 | 0.00 | 0.00 | 0.00 | 0.00 | 0.00 | 0.00 | 0.00 | 0.00 |
| 0.750 | 0.00 | 0.00 | 0.00 | 0.00 | 0.00 | 0.00 | 0.00 | 0.00 | 0.00 | 0.00 | 0.00 | 1.00 |
| 1.500 | 0.00 | 0.00 | 0.00 | 0.00 | 0.00 | 0.00 | 0.00 | 0.00 | 0.00 | 0.00 | 1.27 | 2.47 |
| 3.125 | 0.00 | 0.00 | 1.10 | 2.17 | 0.00 | 0.00 | 0.00 | 2.62 | 0.00 | 2.66 | 5.26 | 5.47 |
| 6.250 | 0.00 | 0.00 | 1.35 | 4.01 | 0.00 | 0.00 | 0.00 | 1.93 | 0.00 | 2.66 | 4.41 | 5.47 |
| 12.500 | 0.00 | 0.00 | 1.42 | 4.62 | 0.00 | 0.00 | 1.08 | 2.09 | 1.61 | 4.05 | 5.56 | 5.47 |
| 25.000 | 0.00 | 0.00 | 1.87 | 5.47 | 0.00 | 0.00 | 1.31 | 4.01 | 2.14 | 5.56 | 5.56 | 5.47 |
| 50.000 | 5.70 | 5.56 | 5.56 | 5.47 | 3.84 | 4.72 | 5.56 | 5.47 | 5.70 | 5.56 | 5.56 | 5.47 |
| 100.00 | 5.70 | 5.56 | 5.56 | 5.47 | 4.43 | 5.56 | 5.56 | 5.47 | 5.70 | 5.56 | 5.56 | 5.47 |

**Table 2**

| Activity of antiseptics on *P. aeruginosa* | | | | | | | | | | | | |
|---|---|---|---|---|---|---|---|---|---|---|---|---|
| | A | | | | B | | | | 1 | | | |
| | 30" | 1' | 2' | 5' | 30" | 1' | 2' | 5' | 30" | 1' | 2' | 5' |
| 0.250 | 0.00 | 0.00 | 0.00 | 0.00 | 0.00 | 0.00 | 0.00 | 0.00 | 0.00 | 0.00 | 0.00 | 0.00 |
| 0.500 | 0.00 | 0.00 | 0.00 | 0.00 | 0.00 | 0.00 | 0.00 | 0.00 | 0.00 | 0.00 | 0.00 | 0.00 |
| 0.750 | 0.00 | 0.00 | 0.00 | 0.00 | 0.00 | 0.00 | 0.00 | 0.00 | 0.00 | 0.00 | 0.00 | 0.00 |
| 1.500 | 0.00 | 0.00 | 0.00 | 0.00 | 0.00 | 0.00 | 0.00 | 0.00 | 0.00 | 0.00 | 0.00 | 1.67 |
| 3.125 | 0.00 | 0.00 | 0.00 | 0.00 | 0.00 | 0.00 | 0.00 | 0.00 | 0.00 | 0.00 | 0.00 | 2.66 |
| 6.250 | 0.00 | 0.00 | 0.00 | 0.00 | 0.00 | 0.00 | 0.00 | 0.00 | 0.00 | 0.00 | 2.13 | 3.87 |
| 12.500 | 0.00 | 0.00 | 1.15 | 1.93 | 0.00 | 0.00 | 0.00 | 1.76 | 1.34 | 2.11 | 3.20 | 5.01 |
| 25.000 | 2.67 | 2.8 | 2.56 | 3.82 | 0.00 | 1.25 | 1.99 | 3.63 | 1.93 | 2.80 | 4.30 | 5.61 |
| 50.000 | 5.67 | 5.65 | 5.56 | 5.61 | 2.26 | 4.33 | 5.56 | 5.61 | 3.54 | 5.65 | 5.56 | 5.61 |
| 100.00 | 5.67 | 5.65 | 5.56 | 5.61 | 2.50 | 5.65 | 5.56 | 5.61 | 3.77 | 5.65 | 5.56 | 5.61 |

**Table 3**

| Activity of antiseptics on *C. albicans* | | | | | | | | | | | | |
|---|---|---|---|---|---|---|---|---|---|---|---|---|
| | A | | | | B | | | | 1 | | | |
| | 30" | 1' | 2' | 5' | 30" | 1' | 2' | 5' | 30" | 1' | 2' | 5' |
| 0.250 | -0.02 | 0.05 | 0.05 | 0.01 | 0.06 | 0.09 | 0.09 | 0.01 | 0.18 | 0.07 | 0.21 | 0.22 |
| 0.500 | 0.08 | 0.00 | 0.00 | 0.02 | 0.27 | 0.79 | 0.47 | 0.50 | 0.18 | 0.31 | 0.43 | 0.56 |
| 0.750 | 0.17 | 0.12 | 0.06 | 0.33 | 0.26 | 0.25 | 0.29 | 0.43 | 0.22 | 0.33 | 0.55 | 1.12 |
| 1.500 | 0.51 | 0.78 | 2.12 | 2.58 | 0.24 | 0.51 | 1.24 | 2.52 | 0.52 | 1.18 | 2.32 | 3.24 |
| 3.125 | 0.44 | 2.52 | 3.35 | 3.88 | 1.26 | 2.73 | 4.35 | 4.35 | 2.07 | 3.75 | 4.35 | 4.35 |

(continued)

| Activity of antiseptics on *C. albicans* | | | | | | | | | | | | |
|---|---|---|---|---|---|---|---|---|---|---|---|---|
| | **A** | | | | **B** | | | | **1** | | | |
| | 30" | 1’ | 2’ | 5’ | 30" | 1’ | 2’ | 5’ | 30" | 1’ | 2’ | 5’ |
| 6.250 | 1.60 | 2.76 | 4.35 | 4.35 | 2.93 | 4.35 | 4.35 | 4.35 | 2.76 | 4.35 | 4.35 | 4.35 |
| 12.500 | 2.77 | 4.35 | 4.35 | 4.35 | 2.45 | 4.35 | 4.35 | 4.35 | 4.37 | 4.35 | 4.35 | 4.35 |
| 25.000 | 3.68 | 4.35 | 4.35 | 4.35 | 4.37 | 4.35 | 4.35 | 4.35 | 4.37 | 4.35 | 4.35 | 4.35 |
| 50.000 | 3.68 | 4.35 | 4.35 | 4.35 | 4.37 | 4.35 | 4.35 | 4.35 | 4.37 | 4.35 | 4.35 | 4.35 |
| 100.00 | 4.37 | 4.35 | 4.35 | 4.35 | 4.37 | 4.35 | 4.35 | 4.35 | 4.37 | 4.35 | 4.35 | 4.35 |

As is evident from the results of activity tests shown above, the antiseptic according to the invention, which is distinguished by freedom from alcohol and the selection of particular non-ionic surfactants, is highly active, the antimicrobial activity of the antiseptic according to the invention being at least as good and in some cases better than that of the comparison antiseptics.

B: Preparation of isotonically adjusted antiseptics

Antiseptic 2 had the following composition (details in % by weight):

| **Component** | **Antiseptic 2** |
|---|---|
| Octenidine dihydrochloride | 0.10 |
| Plantacare® 1200 (50% strength*) | 0.60 |
| Sodium gluconate | 0.40 |
| Glycerol (85% strength*) | 0.50 |
| Sodium chloride | 0.65 |
| Completely demineralized to water | 100.00 |
| * percentage by weight in water | |

Antiseptic 2 is a clear solution which remains clear even on storage for 60 months at 8°C to 40°C.

Antiseptic 2 was diluted in the volume ratio 1:1 with isotonic saline solution (0.9% strength NaCl solution). The resulting formulation antiseptic 3 thus has the following composition (details in % by weight):

| **Component** | **Antiseptic 3** |
|---|---|
| Octenidine dihydrochloride | 0.05 |
| Plantacare® 1200 (50% strength*) | 0.30 |
| Sodium gluconate | 0.20 |
| Glycerol (85% strength*) | 0.25 |
| Sodium chloride | 0.325 |
| Isotonic saline solution | 50.00 |
| Completely demineralized to water | 100.00 |
| * percentage by weight in water | |

Antiseptic 3 is a clear solution which remains clear even after storage for 60 months at temperatures from 8°C to 40°C.

C: Activity of antiseptic (isotonically adjusted using NaCl)

| **S. aureus (100%)** | | | |
|---|---|---|---|
| | Antiseptic 1 | Antiseptic 2 | Comparison antiseptic A |
| 30" | 2.52 | 2.52 | 4.00 |
| 1' | 5.00 | 4.10 | 3.92 |
| 2' | 5.18 | 5.18 | 5.18 |
| 3' | 5.00 | 5.00 | 5.00 |
| 5' 15' | 5.08 | 5.08 | 5.08 |
| | 5.20 | 5.20 | 5.20 |

| **S. aureus (50%)** | | | |
|---|---|---|---|
| | Antiseptic 1 | Antiseptic 2 | Comparison antiseptic A |
| 30" | 2.22 | 2.30 | 0.97 |
| 1' | 4.52 | 3.00 | 1.64 |
| 2' | 5.18 | 3.70 | 4.00 |
| 3' | 5.00 | 5.00 | 5.00 |
| 5' | 5.08 | 5.08 | 5.08 |
| 15' | 5.20 | 5.20 | 5.20 |

| **P. aeruginosa (100%)** | | | |
|---|---|---|---|
| | Antiseptic 1 | Antiseptic 2 | Comparison antiseptic A |
| 30" | 2.63 | 1.03 | 3.18 |
| 1' | 5.41 | 1.52 | 5.41 |
| 2' | 5.34 | 2.44 | 5.34 |
| 3' | 5.38 | 3.34 | 5.38 |
| 5' | 5.18 | 5.18 | 5.18 |
| 15' | 5.08 | 5.08 | 5.08 |

| **P. aeruginosa (50%)** | | | |
|---|---|---|---|
| | Antiseptic 1 | Antiseptic 2 | Comparison antiseptic A |
| 30" | 3.26 | 1.09 | 1.19 |
| 1' | 3.87 | 1.17 | 1.64 |
| 2' | 4.44 | 1.22 | 2.14 |
| 3' | 4.90 | 1.39 | 3.11 |
| 5' | 5.18 | 1.30 | 3.29 |
| 15' | 5.08 | 3.72 | 5.08 |

| **C. albicans (100%)** | | | |
|---|---|---|---|
| | Antiseptic 1 | Antiseptic 2 | Comparison antiseptic A |
| 30" | 4.40 | 0.91 | 4.70 |
| 1' | 4.70 | 1.21 | 4.70 |
| 2' | 4.78 | 3.26 | 4.78 |
| 3' | 4.30 | 3.70 | 4.30 |
| 5' | 4.48 | 4.48 | 4.48 |
| 15' | 4.48 | 4.48 | 4.48 |

| **C. albicans (50%)** | | | |
|---|---|---|---|
| | Antiseptic 1 | Antiseptic 2 | Comparison antiseptic A |
| 30" | 3.62 | 0.00 | 2.80 |
| 1' | 4.70 | 2.55 | 4.70 |
| 2' | 4.78 | 4.78 | 4.78 |
| 3' | 4.30 | 4.30 | 4.30 |
| 5' | 4.48 | 4.48 | 4.48 |
| 15' | 4.48 | 4.48 | 4.48 |

| **Escherichia coli (E. coli)(100%)** | | | |
|---|---|---|---|
| | Antiseptic 1 | Antiseptic 2 | Comparison antiseptic A |
| 30" | 4.78 | 4.78 | 4.78 |
| 1' | 4.90 | 2.60 | 4.90 |
| 2' | 5.08 | 5.08 | 5.08 |
| 3' | 5.00 | 5.00 | 5.00 |
| 5' | 5.00 | 5.00 | 5.00 |

| **E. coli (50%)** | | | |
|---|---|---|---|
| | Antiseptic 1 | Antiseptic 2 | Comparison antiseptic A |
| 30" | 1.82 | 0.71 | 4.78 |
| 1' | 4.06 | 0.99 | 4.90 |
| 2' | 5.08 | 1.57 | 5.08 |
| 3' | 5.00 | 3.24 | 5.00 |
| 5' | 5.00 | 5.00 | 5.00 |

(continued)

| Escherichia coli (E. coli)(100%) | | | | | E. coli (50%) | | | |
|---|---|---|---|---|---|---|---|---|
| | Antiseptic 1 | Antiseptic 2 | Comparison antiseptic A | | | Antiseptic 1 | Antiseptic 2 | Comparison antiseptic A |
| 15’ | 4.78 | 4.78 | 4.78 | | 15’ | 4.78 | 4.78 | 4.78 |
| **E. faecalis (100%)** | | | | | **E. faecalis (50%)** | | | |
| | Antiseptic 1 | Antiseptic 2 | Comparison antiseptic A | | | Antiseptic 1 | Antiseptic 2 | Comparison antiseptic A |
| 30" | 5.08 | 5.08 | 5.08 | | 30" | 5.08 | 4.08 | 2.38 |
| 1’ | 5.08 | 5.08 | 5.08 | | 1’ | 5.08 | 5.08 | 5.08 |
| 2’ | 4.95 | 4.95 | 4.95 | | 2’ | 4.95 | 4.95 | 4.95 |
| 3’ | 5.00 | 5.00 | 5.00 | | 3’ | 5.00 | 5.00 | 5.00 |
| 5’ | 4.95 | 4.95 | 4.95 | | 5’ | 4.95 | 4.95 | 4.95 |
| 15’ | 4.90 | 4.90 | 4.90 | | 15’ | 4.90 | 4.90 | 4.90 |

It results from this that the activity of the phenoxyethanol-free antiseptic according to the invention and of the phenoxyethanol-free antiseptic according to the invention isotonically adjusted using NaCl corresponds approximately to the activity of the phenoxyethanol-containing, non-isotonically adjusted antiseptic.

D: Activity of antiseptics (isotonically adjusted using various sugars)

Various sugars were added to the antiseptics not isotonically adjusted using NaCl for the adjustment of the isotonicity. The quantitative suspension experiment against P. aeruginosa was carried out using the corresponding 50% strength dilutions.

| | Antiseptic 1 | Antiseptic 2 | Comparison antiseptic A | 95 parts by weight of antiseptic 1 + 5 parts by weight of glucose | 95 parts by weight of antiseptic 1 + 5 parts by weight of sorbitol solution (75% by weight) |
|---|---|---|---|---|---|
| 30" | 3.02 | 1.00 | 5.45 | 1.48 | 1.38 |
| 1’ | 4.98 | 1.47 | 5.58 | 1.54 | 1.80 |
| 2’ | 5.32 | 2.02 | 5.32 | 1.73 | 2.23 |
| 3’ | 5.64 | 2.23 | 5.64 | 2.06 | 3.00 |

E: Activity of antiseptics isotonically adjusted (using other additives)

By increasing the additives already present in antiseptic 1, isotonicity was adjusted in antiseptics 4 and 5.

| | Antiseptic 1 | Antiseptic 4 | Antiseptic 5 |
|---|---|---|---|
| Octenidine dihydrochloride | 0.1 | 0.1 | 0.1 |
| Plantacare 2000 | 0.6 | 0.6 | 0.6 |
| Sodium gluconate | 0.4 | 0.4 | 2.8 |
| Glycerol 85% | 0.5 | 2.5 | 0.5 |
| Completely demineralized water | to 100.00 | to 100.00 | to 100.00 |

The antiseptics were investigated with respect to their activity on P. aeruginosa in 50% strength dilutions in hard water:

| | 30" | 1' | 3' | 5' |
|---|---|---|---|---|
| Antiseptic 1 | 3.78 | 3.86 | 5.40 | 5.66 |
| Antiseptic 4 | 3.80 | 3.95 | 5.40 | 5.66 |
| Antiseptic 5 | 4.29 | 4.65 | 5.40 | 5.66 |
| Comparison antiseptic A | 5.65 | 5.26 | 5.40 | 5.66 |

It results from this that the activity of the phenoxyethanol-free antiseptic 1 according to the invention on adjustment of isotonicity, by increasing the content of additives already present in antiseptic 1, is comparable to the activity of the phenoxyethanol-containing, not isotonically adjusted antiseptic.

F: Activity of isotonically adjusted antiseptics at 50% strength dilution

The antiseptics 4 and 5 described in Example E, whose isotonicity was adjusted by an increase in the content of additives already present in antiseptic 1, were investigated with respect to their activity as 50% strength dilutions, on the one hand in hard water and on the other hand in isotonic glucose solution.

| **S. aureus** | **30"** | **1'** | **3'** | **5'** |
|---|---|---|---|---|
| 50% antiseptic 4 in hard water | 3.81 | 4.06 | 4.90 | 5.11 |
| 50% comparison antiseptic A in hard water | 3.30 | 3.88 | 4.60 | 5.11 |
| 50% antiseptic 4 in isotonic glucose solution | 4.78 | 5.18 | 5.11 | 5.15 |
| 50% comparison antiseptic A in isotonic glucose solution | 5.08 | 5.18 | 5.11 | 5.15 |
| **P. aeruginosa** | **30"** | **1'** | **3'** | **5'** |
| 50% antiseptic 4 in hard water | 2.14 | 3.95 | 5.58 | 5.15 |
| 50% comparison antiseptic A in hard water | 5.52 | 5.59 | 5.58 | 5.15 |
| 50% antiseptic 4 in isotonic glucose solution | 4.18 | 5.48 | 5.32 | 5.46 |
| 50% comparison antiseptic A in isotonic glucose solution | 5.38 | 5.48 | 5.32 | 5.46 |
| **S. aureus** | **30"** | **1'** | **3'** | **5'** |
| 50% antiseptic 5 in hard water | 2.70 | 3.27 | 4.90 | 5.11 |
| 50% comparison antiseptic A in hard water | 3.30 | 3.88 | 4.90 | 5.11 |
| 50% antiseptic 5 in isotonic glucose solution | 3.43 | 4.03 | 5.11 | 5.15 |
| 50% comparison antiseptic A in isotonic glucose solution | 5.08 | 5.18 | 5.11 | 5.15 |
| **P. aeruginosa** | **30"** | **1'** | **3'** | **5'** |
| 50% antiseptic 5 in hard water | 1.71 | 3.16 | 4.54 | 4.85 |
| 50% comparison antiseptic A in hard water | 5.52 | 5.59 | 5.58 | 5.15 |
| 50% antiseptic 5 in isotonic glucose solution | 5.38 | 5.48 | 5.32 | 5.46 |
| 50% comparison antiseptic A in isotonic glucose solution | 5.38 | 5.48 | 5.32 | 5.46 |

The 5% strength isotonic glucose solution employed was a commercial product.

It results from this that on dilution of a phenoxyethanol-free antiseptic according to the invention with isotonic glucose solution an increase in activity is achieved, compared with the dilution of a phenoxyethanol-free antiseptic according to the invention with hard water.

G: Turbidity measurements

The following results were obtained after storage of the antiseptics mentioned for 24 hours at +8°C. Here, NTU stands for "nephelometric turbidity unit" (the unit for the measurement at 90° according to the regulations of the EPA, Environmental Protection Agency of the USA). Even an NTU of 20 is discernible as slightly opaque using the naked eye.

| Antiseptic | Turbidity (NTU) |
|---|---|
| Comparison antiseptic A and isotonic saline solution (1:1) | 79.1 |
| Comparison antiseptic A and Ringer's solution (1:1) | 96.4 |
| Antiseptic 4 and isotonic saline solution (1:1) | 0.879 |
| Antiseptic 4 and Ringer's solution (1:1) | 1.26 |

From the results, it is clearly evident that the comparison antiseptic A is more turbid due to addition of isotonic saline or Ringer's solution in the cold than is the case with an antiseptic according to the invention.

## Claims

1. Alcohol-free, aryloxy alcohol-free isotonic antiseptic, **characterized in that** it comprises

   a) 0.01 to 10% by weight of bispyridiniumalkane, in particular octenidine dihydrochloride, and
   b) 0.01 to 20% by weight of non-ionic surfactant selected from alcohol polyalkoxylates, polysorbates and alkyl glycosides,

   and optionally one or more excipients.

2. Antiseptic according to Claim 1, **characterized in that** it comprises

   a) 0.01 to 2% by weight of octenidine dihydrochloride, and
   b) 0.1 to 2% by weight of alkyl glycoside, preferably $C_8$- to $C_{20}$-alkyl polyglucose, more preferably cocoyl polyglucose, lauryl polyglucose or decyl polyglucose or a mixture thereof.

3. Antiseptic according to one of Claims 1 or 2, **characterized in that** the excipient is selected from humectants, thickeners, taste corrigents, refatting agents, buffer substances, antifoam agents and colourants.

4. Use of an antiseptic according to one of Claims 1 to 4 for the manufacture of a skin disinfectant or irrigation solution for antiseptic measures on skin, mucous membrane, wounds or internal organs.

## Patentansprüche

1. Alkoholfreies, aryloxyalkoholfreies, isotonisches Antiseptikum, **dadurch gekennzeichnet, dass** es

   a) 0,01 bis 10 Gew.-% Bispyridiniumalkan, insbesondere Octenidindihydrochlorid, und
   b) 0,01 bis 20 Gew.-% nichtionisches Tensid ausgewählt aus Alkoholpolyalkoxylaten, Polysorbaten und Alkylglykosiden,

   sowie gegebenenfalls einen oder mehrere Hilfsstoff(e) umfasst.

2. Antiseptikum nach Anspruch 1, **dadurch gekennzeichnet, dass** es

   a) 0,01 bis 2 Gew.-% Octenidindihydrochlorid und
   b) 0,1 bis 2 Gew.-% Alkylglykosid, bevorzugt $C_8$- bis $C_{20}$-Alkyl-Polyglucose, bevorzugter Kokoyl-, Lauryl- oder Decylpolyglucose oder deren Mischung, umfasst.

3. Antiseptikum nach einem der Ansprüche 1 oder 2, **dadurch gekennzeichnet, dass** der Hilfsstoff aus Feuchthaltemitteln, Verdickern, Geschmackskorrigentien, Rückfettungsmitteln, Puffersubstanzen, Entschäumern und Farbstoffen ausgewählt ist.

4. Verwendung eines Antiseptikums gemäss einem der Ansprüche 1 bis 3 zur Herstellung eines Hautdesinfektionsmittels oder einer Spüllösung für antiseptische Massnahmen bei Haut, Schleimhaut, Wunden oder inneren Organen.

**Revendications**

1. Antiseptique isotonique exempt d'alcool, exempt de groupes aryloxy, **caractérisé en ce qu'**il comprend

   a) 0,01 à 10 % en poids de bispyridiniumalcane, en particulier le dichlorhydrate d'octénidine, et
   b) 0,01 à 20 % en poids d'un agent tensioactif non ionique choisi parmi les polyalcoxylates d'alcool, les polysorbates et les alkylglycosides,

   et éventuellement un ou plusieurs excipients.

2. Antiseptique selon la revendication 1, **caractérisé en ce qu'**il comprend

   a) 0,01 à 2 % en poids de dichlorhydrate d'octénidine, et
   b) 0,1 à 2 % en poids d'alkylglycoside, de préférence un $C_8$ à $C_{20}$-alkylpolyglucose, de manière particulièrement préférée du cocoylpolyglucose, du laurylpolyglucose ou du décylpolyglucose ou un mélange de ceux-ci.

3. Antiseptique selon l'une quelconque des revendications 1 ou 2, **caractérisé en ce que** l'excipient est choisi parmi les agents mouillants, les épaississants, les correcteurs de goût, les agents relipidants, les tampons, les antimousses et les colorants.

4. Utilisation d'un antiseptique selon l'une quelconque des revendications 1 à 3 pour la préparation d'un désinfectant de la peau ou d'une solution d'irrigation pour des mesures antiseptiques sur la peau, la muqueuse, les plaies ou les organes internes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 2708331 C2
- EP 0411315 A1
- EP 0161425 A
- WO 9820095 A
- DE 19647692 A1
- DE 2708331